# EUROPEAN PATENT APPLICATION

(11) **EP 3 581 934 A1**
(43) Date of publication of application: **18.12.2019**
(21) Application number: 18177797.0
(22) Date of filing: 14.06.2018
(51) Int. Cl.: G01N 33/68

(54) **COMPLEMENT FACTORS FOR DIAGNOSIS OF LOW-GRADE INFECTIONS IN A HUMAN SUBJECT**

(71) Applicant: Otto-von-Guericke-Universität Magdeburg, 39106 Magdeburg (DE); Universita Degli Studi di Trieste, 34127 Trieste (IT)
(72) Inventor: BERTRAND, Jessica, 39112 Magdeburg (DE); LOHMANN, Christoph, 39110 Magdeburg (DE); MÄRTENS, Nicole, 31224 Peine (DE); MEINSHAUSEN, Ann-Kathrin, 39108 Magdeburg (DE); MACOR, Paolo, 34127 Trieste (IT)
(74) Representative: Kailuweit & Uhlemann Patentanwälte Partnerschaft mbB

(57) **Abstract**

The invention relates to a biomarkerfor diagnosis of low-grade infections in a human subject and a method for diagnosis of low-grade infections in a human subject.

## Description

The present invention relates to a method for diagnosis of low-grade infections, a biomarker for use in a method for diagnosis of low-grade infections.

Low-grade infections are a therapeutic challenge. They are difficult to diagnose and extensive to treat. The actual incidence of low-grade infections is unknown but there is evidence in the current literature that low-grade infections are more common than previously assumed. An accurate diagnosis as well as the identification of the pathogen are required for a successful therapy.

Problems especially arises after surgery, e.g. implant surgery, due to missing clinical signs of infection. A proper therapeutic treatment is sufficient in order to avoid surgical revisions, e.g. in case of implant surgery.

Shoulder arthroplasty is a rapidly growing procedure amongst all total joint replacements with a seven-fold increase predicted for the next 15 years (Deore 2017). Today, various different types of shoulder prostheses for different indications and individual anatomical conditions exist. However, the design of these prostheses can be distinguished in two basic biomechanical principles, the anatomic and the reversed shoulder implants.

Due to the increasing number of primary shoulder arthroplasties, the number of septic and aseptic surgical revisions is a rising challenge in shoulder surgery. The rate of infection in shoulder endoprostheses is approximately 1% and is comparable to the infection rate of other joints (Padegimas 2015). The most frequently detected pathogens in shoulder TJA are *Propionibacterium acnes* (*P. acnes*), *Staphylococcus epidermidis* (*S. epidermidis*) and *Staphylococcus aureus* (*S. aureus*) (Piper 2009). The causes of infection can be a hematogenic infection (pneumonia, urinary tract infection, dental sinuses) or the intra- or perioperative infection which can appear as early or late infections. Clinically, an early periprosthetic joint infection (PJI) is accompanied by onset of pain, loss of function and other signs of inflammation such as fever, wound healing disorders or the development of local erythema. Furthermore, the presence of a systemic reaction is indicated by increased systemic inflammation parameters such as WBC and CRP values (Parvizi 2014). The late infection, however, is often more difficult to diagnose, because of the lack of a systemic inflammation due to the formation of a biofilm. Sometimes radiological detectable periprosthetic radiolucent lines indicate the failure of a secure fixation of the implant under low-grade septic complications. The microbiological analysis of the synovial fluid, however, detects low-grade infection cases only at very late stages, when the biofilm already produces planktonic bacteria. A second surgical intervention using a spacer implant, consisting of antibiotics and bone cement, is in most cases inevitable. Since low grade infections are difficult to diagnose at early stages and the late diagnosis make appropriate treatment of the infection without explantation of the prosthesis impossible, there is a need for biomarkers to diagnose the infection at an early time point. To develop biomarkers for the early diagnosis of low grade infection the understanding of different pathways activated during the infection is of utmost importance.

The detection of α-defensin has been proposed to be a marker for PJI (Bingham 2014, Ganz 1985, White 1995). The α-defensin protein is a 2-6 kDa antimicrobial peptide, which is predominantly activated by Gram-negative and Gram-positive bacteria. It is secreted by neutrophils and macrophages and is able to bind pathogens in the synovial fluid and impede cell wall synthesis (Ganz 1985, White 1995). However, there have been reports of false positive test results in case of adverse tissue reactions (Eriksson 2018).

Another important component of the immune response to bacterial infection is the complement system (Schifferli 1986). The main purpose of the complement system is the destruction of foreign or dead cells, activation of the immune defense cells and the opsonization of pathogens (Mevorach 1998). Therefore, the activation of the complement system is predominantly active in the early infection phase (Huber-Lang 2006, Langlois 1988). The system recognizes foreign structures activating three different pathways, that converge to the common component C3; terminal common pathway is initiated with C5 being cleaved into C5a and C5b. C5b starts the formation of the membrane attack complex (MAC) by recruiting C6, C7, C8 and C9. MAC is the cytolytic endproduct of the terminal complement cascade resulting in osmotic lysis and thereby cell death (Dunkelberger 2010).

The presence of macrophages in tissue biopsies has been proposed to be an indicator for septic complications, as they are part of the non-specific immune response by removing pathogens via phagocytosis and also part of the adaptive immune response by recruiting other immune cells. Immunostaining for CD68 shows monocytes and macrophages presence, as a first hint of inflammatory tissue response (Parwaresch 1986, Saito 1991).

The hypothesis of the study was that the terminal complement pathway in combination with α-defensin provides better evidence for discrimination between aseptic loosening and PJI of total shoulder arthroplasties. To test this hypothesis, we investigated aseptic and septic tissues of shoulder endoprostheses revision surgeries, with regard to the design of the shoulder implant, patient characteristics, bacterial diagnostic and the proposed biomarkers.

Therefore, it is an object of the present invention to provide a biomarker that allow a diagnosis for low-grade infections and a method for such diagnosis.

The objective is solved by a biomarker and a method for diagnosis of low-grade infections according to the present invention.

In a first aspect the invention provides a biomarker for diagnosis of low-grade infections.

The biomarker is selected from a group consisting of factors of the complement system.

In certain embodiments the biomarker is selected from a group consisting of complement factors C3, C5 and C9. Preferably, the biomarker is complement factor C9.

Surprisingly, it was found that biomarker allows a separation of septic from aseptic tissue with a predictive specificity of 100%.

In a further aspect the invention provides a method for diagnosis of low-grade infections in a human subject.

The method comprises the steps of:
- providing a sample of the human subject,
- ex vivo detection of the biomarker according to the present invention within the sample,
- correlation of the result with the low-grade infection disease state.

Preferably the sample is a tissue sample, e.g. obtained by biopsy or a blood sample or plasma sample.

In certain embodiments the detection of the biomarker is performed by immunohistochemically staining, enzyme-linked immunosorbent assay (ELISA), Polymerase-chain-reaction (PCR) Enzyme-Linked ImmunoSpot (ELISPOT), Enzyme multiplied immunoassay technique (EMIT), Immunofluorescence, radiobinding assay, radioimmunoassay (RIA), Western-Blot or Fluorescence in situ hybridization (FISH).

In certain embodiments, the correlation of the obtained results with the low-grade infection disease state is performed by determination of the presence of the biomarker within the sample. In case that the detection of the biomarker within the sample is positive, a low-grade infection is diagnosed and further steps in view of therapeutic treatment can be performed.

With the biomarker and the method for diagnosis of low-grade infections in a human subject, a fast and specific diagnosis is provided which allows an accelerated therapeutic treatment and a reduce in surgery revisions.

The invention will be explained in more detail with the aid of the following figures and embodiments without limiting the invention to them.

For the purpose of illustrating aspects of the present invention, there are depicted in the drawings certain embodiments of the invention. However, the invention is not limited to the precise arrangements and instrumentalities of the embodiments depicted in the drawings. Further, as provided, like reference numerals contained in the drawings are meant to identify similar or identical elements.

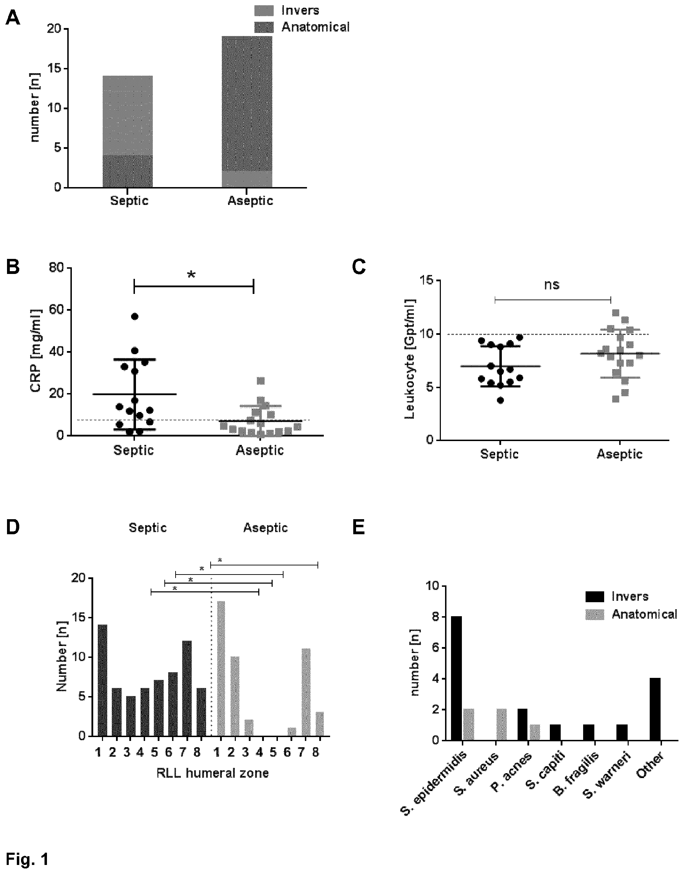

Fig. 1 depicts the characterization of septic and aseptic shoulder prostheses. (A) 33 implants were included in the study (14 septic and 19 aseptic). (B) CRP value [mg/I] of the septic (black circle) and aseptic (grey square) was significantly increased in septic samples. The pathological threshold of 5 mg/l is indicated as red dotted line. (C) The WBC value (Gpt/I) is not changed between septic and aseptic group. Normal range of 10 Gpt/I is given as red dotted lines (D) RLL of the humeral zones were analyzed by using the Neer zones (Neer et al., 1982). The septic group exhibited increased RLL compared to the aseptic group. (E) Prevalence of the most frequent pathogens in the reverse and anatomical shoulder prostheses;

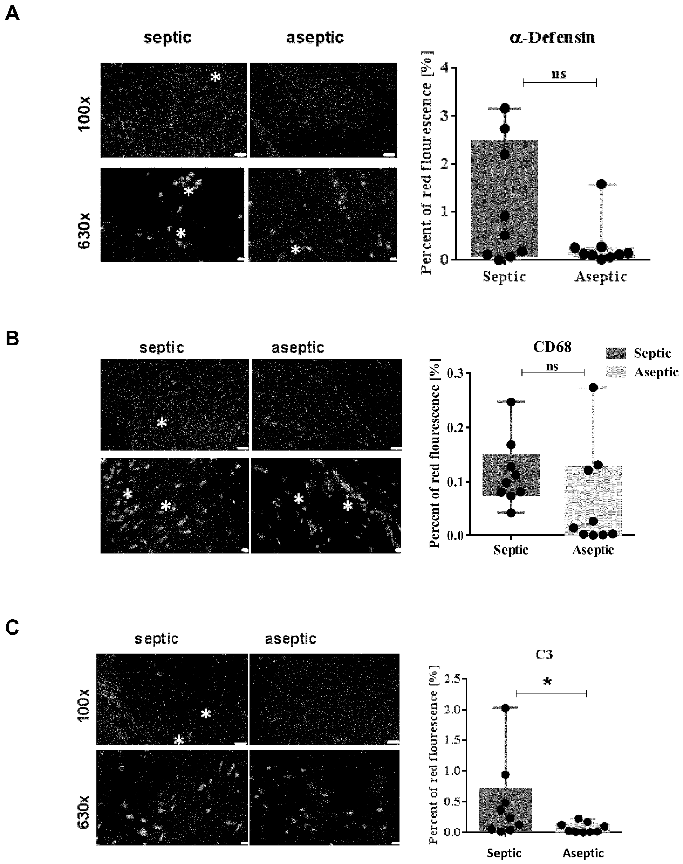

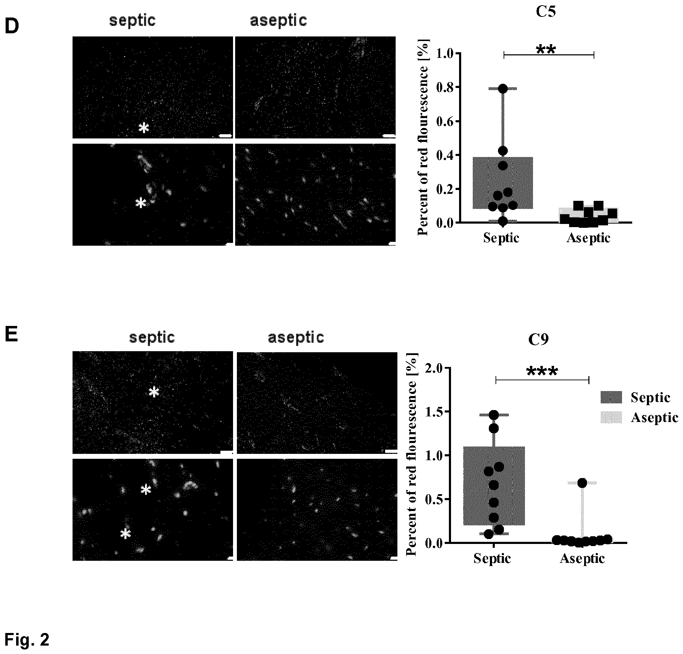

Fig. 2 depicts the local inflammatory response in septic and aseptic periprosthetic tissue. Representative immunohistochemical stainings of the periprosthetic tissue of septic and aseptic patients. α-defensin (A), macrophages (CD68) (B). Terminal complement pathway (C- E) (N = 9; n = 27). The values indicate the percentage of positive staining given as median of 3 images per patient. Two different magnifications (100x, 400x) are given. In the 100x magnification, the scale bar is 100 µm, while the scale bar in the 630x magnification is 2 µm (white bars). (n = 3; N = 9).

### Patients

33 consecutive shoulder revision surgeries due to aseptic and septic revision reasons were included between February 2011 and April 2016 (IRB No. 150/ 12, Institutional Review Board of the Medical School, Otto-von-Guericke University, Magdeburg). The demographic data of all patients (age at surgery, implantation time, radiologically detected radiolucent lines (RLL), number of previous surgeries and comorbidities) were recorded (Table 1). Prior to surgery, serum levels of C-reactive protein (CRP, mg/l) and white blood cells (WBC, Gpt/I) were determined. Infections were identified according to MSIS criteria and IDSA criteria (Osmon 2013, Parvizi 2014).

### Histology and immunohistochemically staining

The periprosthetic tissues were fixed overnight in 4% paraformaldehyde. The tissue was embedded in paraffin and cut in 4 µm sections. Immunohistochemical staining was performed using the following antibodies: α-defensin (Acris, OriGene Technologies Inc., Rockville, United States), CD68 (Santa Cruz Biotechnology, Inc. Europe), C3/C5/C9 (Quidel, San Diego, CA, USA). Corresponding IgG antibodies were used as isotype control for the respective stainings. The area of red immunofluorescence was calculated as percentage of the picture and was analyzed using ImageJ 1.5 (NIH, USA) in comparison to the isotype control staining.

### Microbiologic diagnostic

Periprosthetic tissue samples were cut into pieces and mechanically homogenized on Ultra-Turrax Drive control Dispergierer (IKA®-Werke GmbH & Co. KG, Germany) at 6,000 rpm for 2 min in interval direction change. Briefly, the homogenized samples were inoculated on agar plates: columbia agar with 5% sheep blood (Becton Dickinson, Heidelberg, Germany), chocolate agar and Schaedler agar (Oxoid, Munich, Germany) under aerobic conditions with 5% CO2 and anaerobically at 35±1 °C. Additionally, the samples were inoculated in thioglycolate and Schaedler broth (bioMérieux, Marcy L'Etoile, France) at 35±1 °C for 14 days. The identification of pathogens was performed by MALDI-TOF MS (VITEK® MS, bioMerieux, Marcy L'Etoile, France).

### Radiographic Analysis

For the evaluation of periprosthetic bone resorption the X-rays were analyzed for the location and extent auf radiolucent lines (RLL) around the implant (Sanchez-Sotelo 2001). To -determine the degree of implant loosening, the humeral component of the shoulder was subdivided into eight radiological zones (Neer 1982), the glenoid was not investigated due to the relatively small number of aseptic revers implants.

### Statistical analysis

Statistical analysis was performed using GraphPad Prism V7 (GraphPad Software, San Diego, USA) and SPSS (Chicago, USA). Medcalc (MedCalc Software bvba, Ostend, Belgium) was used for calculation of C9 staining predictive sensitivity and specificity. The Plots and bar charts show Median with SD. For the test of statistical significance we use the Mann-Whitney-U-Test, the statistical significance is indicated as *P < 0.01, **P < 0.05 and ***P<0.1.

### Demographics and patient characteristics

33 patients undergoing a shoulder revision were included. Microbiological diagnostic indicated PJI in 14 patients as reason for revision. 19 patients were considered as aseptic revisions (Fig. 1 A), due to rotator cuff insufficiency and instability (9/19), glenoid component loosening (1/19), glenoid erosion (2/19), prosthesis dislocation (3/19), periprosthetic fractures (2/19) and humeral osteolysis (2/19).

The septic group of 14 patients was subdivided into 10 patients with reverse and 4 with anatomical shoulder implants, whereas the aseptic group included 2 reverse and 17 anatomical implants (Table 1, Fig 1A). Altogether, in total 19 (57.6 %) patients underwent aseptic surgery and 14 (42.4 %) patients were diagnosed with a bacterial infection. Interestingly, 71.4 % of the septic revision surgeries received a revision of a reverse shoulder prostheses, while only 28.6% of the patients with anatomical implants showed a septic complication. The average age of the patients at the time of surgery was similar in both groups (aseptic: 73.3 years and septic: 72.1 years). The implantation time before revision surgery was approximately 3 years (aseptic: 3.44 ± 4.84 and septic: 2.19± 2.28 years) (Table 1).

The number of previous surgeries before revision surgery of the respective joint is presented in detail in Table 1. The number of previous surgeries in the septic TSA group was greater than in the aseptic TSA group, with 9 out of 11 patients having at least 1 or 2 previous shoulder surgeries and 1 patient having 3 previous surgeries of the shoulder. In the aseptic patient group 5 patients had 1 or at least to 2 prior operations. The remaining 14 patients had no previous operation before revision surgery.

Comorbidities of the patients are presented in Table 2. Patients with septic complications had more often diabetes and renal insufficiencies, whereas patients with aseptic revision showed more neurological comorbidities like Parkinson's disease or polyneuropathies.

To evaluate the presence of systemic inflammation, the C-reactive protein (CRP) value and the WBC count were analyzed in the blood of all patients.

When comparing the CRP value (Fig. 1 B) between the aseptic (4.55 mg/l) and the septic (13.10 mg/l) patient group, the septic group showed a higher variability in CRP values than the aseptic group (p ≤ 0.0138).

No difference, however, was observed regarding the WBC count of septic and aseptic patients (Fig. 1 C). The aseptic group showed the average WBC count of 8.2 Gpt/I, while the average WBC value of the septic group was at 6.6 Gpt/I. As the normal maximum value of WBC is given as 10 Gpt/I, no pathological elevation of WBC was observed in both groups (p= 0.157).

The evaluation of radiolucent lines (RLL) in the X-ray images showed clear differences in the bone resorption pattern between septic and aseptic cases (Fig. 1 D). The X-rays of the septic patient group exhibited RLL in all 8 Neer zones of the humerus. Interestingly, the aseptic group mainly exhibited loosening in the zones 1 and 7 of the humerus. The other RLL zones were mainly not affected in the aseptic group, indicated by the fact that the zones 4, 5, 6 and 8 were statistically more frequently found in the septic group compared to the aseptic (zone 4 p= 0.03, zone 5 p= 0.01, zone 6 p= 0.02, zone 8 p= 0.04). Because of the very low number of aseptic and reverse cases (n=2), we did not investigate the glenoid loosening.

The identification of bacterial strains are summarized in Fig. 1E. Interestingly, S. epidermidis showed the highest frequency in the reverse shoulder implants (36.4 %), while S. aureus (9.1 %) was only found in the anatomical prostheses. The reverse shoulder implants showed a higher variety of different Staphylococcus strains compared to the anatomical prostheses. Other bacteria, such as S. capiti (4.5 %), B. fragilis (4.5 %) and S. warneri (4.5 %) were only found in small numbers on reverse, but not in the anatomical group. While P. acnes was found in the anatomical (4.5 %) and the reverse (9.1 %) shoulder implants.

### Distribution of septic marker proteins in periprosthetic revision tissue

As α-defensin staining has been proposed to be a marker for an infected tissue, we stained septic and aseptic periprosthetic revision tissue for the tissue presence and distribution of this marker protein (Fig. 2A). We found both tissue types to be positive for α-defensin, with no significant difference between the septic and the aseptic group (septic: 0.5171% ± 0.4187, aseptic: 0.1307 ± 0.1631 p= 0.2224).

To further analyze the tissue response in septic revision tissue, we assessed the presence of macrophages as inflammatory marker cells using CD68 staining (Fig 2B). Again, we found macrophages to be present in septic as well as in aseptic tissue and the quantification of the fluorescence showed no difference between septic and aseptic tissue (septic: 0.097 ± 0.02, aseptic: 0.014 ± 0.03 p= 0.1135).

The terminal pathway of the complement system is a key component of the host defense against bacteria. We investigated this pathway using antibodies for C3, C5 and C9 to evaluate their tissue presence (Fig. 2C-E). We found an increase deposition of all studied complement components in septic tissues. C3, as the first common activated component, was significantly increased in the septic tissue (septic: 0.23 ± 0.2175 and aseptic: 0,029 %± 0.027, p= 0,031) (Fig. 2C). The statistical difference increased with C5, as the following component starting the terminal pathway (septic: 0.16 ± 0.08 and aseptic: 0,022 %± 0.013, p= 0,005) (Fig. 2D). Interestingly, C9, as the furthest downstream component of this pathway, distinguished with a specificity of 100% and a sensitivity of 88.89% between septic and aseptic tissue (septic: 0.662 ± 0.161 and aseptic: 0.025 %± 0.073, p= 0.0008) (Fig. 2E).

In this study, 33 consecutive total shoulder revisions were analyzed with respect to reasons for revisions, radiological detectable radiolucent lines, patient characteristics as well as microbiological diagnostic. The aim of the study was to determine if biomarkers such as α-defensin and the terminal complement pathway have the potential to discriminate between septic and aseptic total shoulder arthroplasty loosening. Therefore, aseptic (57.6%) or septic (42.4%) periprosthetic revision tissues were tested. The findings of our study suggest that particularly the deposition of the terminal complement component C9 discriminates with an extremely high sensitivity of 100% between PJI and aseptic loosening in shoulder endoprostheses.

Comorbidities, including diabetes, renal diseases or heart failure, are known to increase the risk of periprosthetic infection (Baek, 2014, Bozic 2012, Daines 2015, Phillips 2013, Pulido 2008). We also observed an increased number of patients with diabetes and renal insufficiency in our septic cohort; however, we found also patients with these comorbidities in the aseptic cohort and septic patients without these comorbidities. Furthermore, the risk of PJI increases with each revision surgery by about 10% (Fehring 2000, Goldberg 1988, Smucny 2015). In our cohort, there was also an increase in the number previous surgeries in the septic subgroup, which might explain partially the increased number of PJI. Again, there were also patients with PJI, who did not have a revision surgery before infection and some of these patients did not have any relevant comorbidities. However, these factors clearly contributed to the finding of an increased number of septic complications with a reversed design, but did not explain the increased number completely.

According to their biomechanical principles, one difference between the anatomical and reverse prostheses is that screws are used for fixation for the glenoid component in the reverse shoulder prostheses. It has been shown that in wide surface grooves of the screws, the contact area of the bacterium to the material is increased and the adhesion energy is enhanced, which allows the bacteria to adhere better on the surface (G S 1999, Roosjen 2005, Sénéchal 2004, Whitehead 2004). In line with these observations, it has been shown that the one type of stemless shoulder prostheses, which was fixed with a porous coated hollow screw, is more prone to infection compared to other anatomic shoulder prosthetic designs (Johansson 2017). This might be a reason for the increased infection rate of screw fixed prostheses.

We also observed a difference in the bacterial spectrum between anatomical and reverse shoulder implants. This difference might be explained by the fact that some strains prefer to adhere to smooth while other bacteria colonize better on rough surfaces (Yamauchi. 1990) Interestingly, it has been proven that S. epidermidis shows a significantly increased adhesion on rougher surfaces (Verran 2005, Wassmann 2017), giving rise for an explanation for the higher frequency of *S. epidermidis* infection of reverse prostheses. Furthermore, *S. epidermidis* was mostly found in co-colonization with other bacteria, whereas *S. aureus* was mostly found as a monoculture. Previous studies show that *S. aureus* exhibits a higher virulence than *S. epidermidis* making a co-colonization thereby impossible (Gill, 2005, Maki 1973).

One key problem of low-grade PJI is the early diagnosis. To test whether an increase in markers of a systemic inflammation could be observed in the septic patients, we analyzed serum CRP value and WBC counts. We observed an increase in CRP to 19.84 mg/l, which was statistically significant. However, the predictive value of CRP measurement to identify infections of endoprostheses has been described to be low (Pohlig, 2017, Sanzén 1997) indicating that especially the low-grade infection is a local phenomenon with very low systemic involvement. The gold standard for the diagnosis of low-grade PJI therefore, is at the moment the examination of an arthroscopic tissue biopsy (Pohlig.2017)

We stained for the presence of macrophages in the septic and aseptic tissue using CD68 as a marker. There was no significant difference in macrophage presence in septic periprosthetic tissue compared to aseptic revision tissue. The presence of macrophages in aseptic tissue might be attributed to the presence of wear particles, which are phagocytosed by macrophages (Ito 2004, John Looney 2006, Purdue 2007, Sargeant 2006).

Another proposed biomarker for bacterial infection is α-defensin (Deirmengian 2015). Using immunohistological stainings we also found α-defensin in the periprosthetic tissue of septic as well as the aseptic samples. The quantitative analysis of the immunofluorescence revealed no significant increase in α-defensin in septic tissues compared to the aseptic tissue. Therefore, the predictive value of α-defensin, therefore, as a biomarker for infection in our shoulder cohort was low. This finding might be explained by other studies showing that the presence of metal wear particles can also induce the expression of α-defensin (Bingham 2014, Deirmengian. 2014). This might be a reason for the positive α-defensin staining in the aseptic tissues.

One main part of the immune system involved in the host defense against bacteria is the complement system (Atkinson 2006, Morgan 1998, Rickling. 2007) To investigate its activation, we stained for three different components (C3, C5 and C9) of the terminal part of the complement pathway in septic and aseptic tissue. C3 is the pivotal component of all the three pathways of activation while C5 and, in particular, C9 are members of the common late pathway and their deposition indicate a terminal part of the cascade. The quantitative analysis showed a significant increase of all complement factors in the septic group, with C9 being able to detect septic tissue with a high specificity and sensitivity. In contrast to α-defensin, we found almost no presence of complement factors in non-septic tissue, making the activation of the complement system a highly specific marker for infection (Bengtson 1988, Heideman 1988, Zilow 1997).

Furthermore, we tested the sensitivity of C9 as biomarker at other localizations of PJI. We found the same sensitivity for septic hip tissue and septic knee tissue. We included 40 aseptic hip tissues and 40 septic hip tissues, as well as 35 septic knee tissues and 40 aseptic knee tissues with the same result. We did not observe any reduction of sensitivity when wear debris was present in the tissue, as well as in tissue of crystalopathy patients.

### Cited literature

1. Atkinson JP, Frank MM. Bypassing complement: evolutionary lessons and future implications. J Clin Invest. 2006. 2006;116(5):2004-2007. doi:10.1172/JCI28622.ment
2. Baek S-H. Identification and preoperative optimization of risk factors to prevent periprosthetic joint infection. World J. Orthop. [Internet]. 2014;5(3):362. Available from: http://www.wjgnet.com/2218-5836/full/v5/i3/362.htmdoi:10.5312/wjo.v5.i3.362
3. Bengtson A, Heideman M. Anaphylatoxin Formation in Sepsis. Arch. Surg. 1988;123(5):645-649. doi:10.1001/archsurg.1988.01400290131023
4. Bingham J, Clarke H, Spangehl M, Schwartz A, Beauchamp C, Goldberg B. The Alpha Defensin-1 Biomarker Assay can be Used to Evaluate the Potentially Infected Total Joint Arthroplasty. Clin. Orthop. Relat. Res. 2014;472(12):4006-4009. doi:10.1007/s11999-014-3900-7
5. Bozic KJ, Lau E, Kurtz S, Ong K, Rubash H, Vail TP, et al. Patient-Related Risk Factors for Periprosthetic Joint Infection and Postoperative Mortality Following Total Hip Arthroplasty in Medicare Patients. J. Bone Jt. Surgery-American Vol. [Internet]. 2012;94(9):794-800. Available from: http://content.wkhealth.com/linkback/openurl?sid=WKPTLP:landingpage&an=00004623-201205020-00004doi:10.2106/JBJS.K.00072
6. Daines BK, Dennis D a, Amann S. Infection Prevention in Total Knee Arthroplasty. J Am Acad Orthop Surg [Internet]. 2015;23(6):356-364. Available from: http://www.ncbi.nlm.nih.gov/pubmed/26001428doi:10.5435/JAAOS-D-12-00170
7. Deirmengian C, Kardos K, Kilmartin P, Cameron A, Schiller K, Parvizi J. Combined measurement of synovial fluid α-Defensin and C-reactive protein levels: highly accurate for diagnosing periprosthetic joint infection. J. Bone Joint Surg. Am. [Internet]. 2014;96(17):1439-45. Available from: http://www.ncbi.nlm.nih.gov/pubmed/25187582doi:10.2106/JBJS.M.01316
8. Deirmengian C, Kardos K, Kilmartin P, Gulati S, Citrano P, Booth RE. The Alpha-defensin Test for Periprosthetic Joint Infection Responds to a Wide Spectrum of Organisms. Clin. Orthop. Relat. Res. [Internet]. 2015;473(7):2229-2235. Available from: http://dx.doi.org/10.1007/s11999-015-4152-xdoi:10.1007/s11999-015-4152-x
9. Deore VT, Griffiths E, Monga P. Shoulder arthroplasty-Past, present and future. J. Arthrosc. Jt. Surg. 2017;5:3-8. doi:10.1016/j.jajs.2017.12.001
10. Dunkelberger JR, Song WC. Complement and its role in innate and adaptive immune responses. Cell Res. [Internet]. 2010;20(1):34-50. Available from: http://dx.doi.org/10.1038/cr.2009.139doi:10.1038/cr.2009.139
11. Eriksson HK, Nordstr J, Gabrysch K, Hailer NP. Does the Alpha-defensin Immunoassay or the Lateral Flow Test Have Better Diagnostic Value for Periprosthetic Joint Infection? A Systematic Review. Clin. Orthop. Relat. Res. 2018;1-8. doi:10.1007/s11999.0000000000000244
12. Fehring TK, Odum S, Calton TF, Mason JB. Articulating versus static spacers in revision total knee arthroplasty for sepsis. The RanawatAward. Clin. Orthop. Relat. Res. [Internet]. 2000;(380):9-16. Available from: http://www.ncbi.nlm.nih.gov/pubmed/11064968doi:10.1097/00003086-200011000-00003
13. G S, I G, EO P, J F. Cell adhesion force microscopy. Proc Natl Acad Sci USA. 1999;96:1999.
14. Ganz T, Selsted ME, Szklarek D, Harwig SS, Daher K, Bainton DF, et al. Defensins. Natural peptide antibiotics of human neutrophils. J. Clin. Invest. 1985;76(4):1427-1435. doi:10.1172/JC1112120
15. Gill SR, Fouts DE, Archer GL, Mongodin EF, Deboy RT, Ravel J, et al. Insights on Evolution of Virulence and Resistance from the Complete Genome Analysis of an Early Methicillin-Resistant Staphylococcus aureus Strain and a Biofilm-Producing Methicillin-Resistant Staphylococcus epidermidis Strain. J. Bacteriol. 2005;187(7):2426-2438. doi:10.1128/JB.187.7.2426
16. Goldberg AL, Kettelhut IC, Furuno K, Fagan JM, Baracos V. Activation of protein breakdown and prostaglandin E2production in rat skeletal muscle in fever is signaled by a macrophage product distinct from interleukin 1 or other known monokines. J. Clin. Invest. 1988;81(5):1378-1383. doi:10.1172/JCl113466
17. Heideman M, Norder-Hansson B, Bengtson A, Mollnes TE. Terminal Complement Complexes and Anaphylatoxins in Septic and Ischemic Patients. Arch. Surg. 1988;123(2):188-192. doi:10.1001/archsurg.1988.01400260068008
18. Huber-Lang M, Sarma JV, Zetoune FS, Rittirsch D, Neff TA, McGuire SR, et al.Generation of C5a in the absence of C3: A new complement activation pathway. Nat. Med. 2006;12(6):682-687. doi:10.1038/nm1419
19. Ito S, Matsumoto T, Enomoto H, Shindo H. Histological analysis and biological effects of granulation tissue around loosened hip prostheses in the development of osteolysis. J. Orthop. Sci. 2004;9(5):478-487. doi:10.1007/s00776-004-0808-1
20. Johansson L, Hailer NP, Rahme H. High incidence of periprosthetic joint infection with propionibacterium acnes after the use of a stemless shoulder prosthesis with metaphyseal screw fixation - a retrospective cohort study of 241 patients propionibacter infections after eclipse TSA. BMC Musculoskelet. Disord. 2017;18(1):1-8. doi:10.1186/s12891-017-1555-8
21. John Looney R, Schwarz E, Boyd A. Periprosthetic osteolysis: an immunologisfs update. Curr. Opin. Rheumatol. - CURR OPIN RHEUMATOL. 2006;18:80-87.
22. Langlois PF, Gawryl MS. Accentuated formation of the terminal C5b-9 complement complex in patient plasma precedes development of the adult respiratory distress syndrome. Am. Rev. Respir. Dis. 1988;138(2):368-375. doi:10.1164/ajrccm/138.2.368
23. Maki D, Goldman D, Rhame F. INfection control in intravenous therapy. Ann. Intern. Med. [Internet]. 1973 Dec 1;79(6):867-887. Available from: http://dx.doi.org/10.7326/0003-4819-79-6-867
24. Mevorach BD, Mascarenhas JO, Gershov D, Elkon KB. Complement-dependent Clearance of. J. Exp. Med. 1998;188(12):2313-2320.
25. Morgan B. The Human Complement System in Health and Disease. Ann. Rheum. Dis. [Internet]. 1998 Oct;57(10):581. Available from: http://www.ncbi.nlm.nih.gov/pmc/articles/PMC1752481/
26. Neer CS, Watson KC, Stanton FJ. Recent experience in total shoulder replacement. J. Bone Joint Surg. Am. [Internet]. 1982;64(3):319-37. Available from: http://www.ncbi.nlm.nih.gov/pubmed/7061549doi:10.1007/978-1-4471-5451-8_81
27. Osmon DR, Berbari EF, Berendt AR, Lew D, ZimmerliW, Steckelberg JM, et al. Diagnosis and management of prosthetic joint infection: clinical practice guidelines by the Infectious Diseases Society of America. Clin. Infect. Dis. [Internet]. 2013;56(1):e1-e25. Available from: isi:000312605500005doi:10.1093/cid/cis803\rcis803 [pii]
28. Padegimas EM, Maltenfort M, Ramsey ML, Williams GR, Parvizi J, Namdari S. Periprosthetic shoulder infection in the United States: Incidence and economic burden. J. Shoulder Elb. Surg. [Internet]. 2015;24(5):741-746. Available from: http://dx.doi.org/10.1016/j.jse.2014.11.044doi:10.1016/j.jse.2014.11.044
29. Parvizi J, Gehrke T. Definition of periprosthetic joint infection. J. Arthroplasty. 2014;29(7):1331. doi:10.1016/j.arth.2014.03.009
30. Parwaresch MR, Radzun HJ, Kreipe H, Hansmann ML, Barth J. Monocyte/macrophage-reactive monoclonal antibody Ki-M6 recognizes an intracytoplasmic antigen. Am. J. Pathol. [Internet]. 1986;125(1):141-51. Available from: http://www.pubmedcentral.nih.gov/articlerender.fcgi?artid=1888444&tool=pmcentrez&re ndertype=abstract
31. Phillips JE, Crane TP, Noy M, Elliott TSJ GR. The incidence of deep prosthetic infections in a specialist orthopaedic hospital. 2013; 1-17.
32. Piper KE, Jacobson MJ, Cofield RH, Sperling JW, Sanchez-Sotelo J, Osmon DR, et al. Microbiologic diagnosis of prosthetic shoulder infection by use of implant sonication. J. Clin. Microbiol. 2009;47(6):1878-1884. doi:10.1128/JCM.01686-08
33. Pohlig F, Mühlhofer HML, Lenze U, Lenze FW, Suren C, Harrasser N, et al. Diagnostic accuracy of arthroscopic biopsy in periprosthetic infections of the hip. Eur. J. Med. Res. 2017;22(1):1-7. doi:10.1186/s40001-017-0246-0
34. Pulido L, Ghanem E, Joshi A, Purtill JJ, Parvizi J. Periprosthetic joint infection: The incidence, timing, and predisposing factors. Clin. Orthop. Relat. Res. 2008;466(7):1710-1715. doi:10.1007/s11999-008-0209-4
35. Purdue PE, Koulouvaris P, Potter HG, Nestor BJ, Sculco TP. The cellular and molecular biology of periprosthetic osteolysis. Clin. Orthop. Relat. Res. 2007;(454):251-261. doi:10.1097/01.blo.0000238813.95035.1b
36. Rickling D, D LJ. Complement-targeted therapeutics. Nat. Biotechnol. 2007;25(11):1265-1275. doi:10.1038/nbt1342.Complement-targeted
37. Roosjen A, Boks NP, Van Der Mei HC, Busscher HJ, Norde W. Influence of shear on microbial adhesion to PEO-brushes and glass by convective-diffusion and sedimentation in a parallel plate flow chamber. Colloids Surfaces B Biointerfaces. 2005;46(1):1-6. doi:10.1016/j .colsurfb.2005.08.009
38. Saito N, Pulford KA, Breton-Gorius J, Masse JM, Mason DY, Cramer EM. Ultrastructural localization of the CD68 macrophage-associated antigen in human blood neutrophils and monocytes. Am. J. Pathol. [Internet]. 1991;139(5):1053-9. Available from: http://www.pubmedcentral.nih.gov/articlerender.fcgi?artid=1886337&tool=pmcentrez&re ndertype=abstract
39. Sanchez-Sotelo J, Cofield RH, Rowland CM. Shoulder hemiarthroplasty for glenohumeral arthritis associated with severe rotator cuff deficiency. J Bone Joint Surg Am. 2001 Dec;83-A(12): 1814-22.
40. Sanzén L, Sundberg M. Periprosthetic low-grade hip infections. Erythrocyte sedimentation rate and C-reactive protein in 23 cases. Acta Orthop. Scand. 1997;68(5):461-465. doi:10.3109/17453679708996263
41. Sargeant A, Goswami T. Pathophysiological aspects of hip implants. J. Surg. Orthop. Adv. 2006;15(2):111-112. doi:424
42. Schifferli JA, Ng YC, Peters DK. The role of complement and its receptor in the elimination of immune complexes. N. Engl. J. Med. [Internet]. 1986;315(8):488-495. Available from: http://www.nejm.org/doi/abs/10.1056/NEJM198608213150805doi:10.1056/NEJM198608 213150805
43. Sénéchal A, Carrigan SD, Tabrizian M. Probing surface adhesion forces of enterococcus faecalis to medical-grade polymers using atomic force microscopy. Langmuir. 2004;20(10):4172-4177. doi:10.1021/la035847y
44. Smucny M, Menendez ME, Ring D, Feeley BT, Zhang AL. Inpatient surgical site infection after shoulder arthroplasty. J. Shoulder Elb. Surg. [Internet]. 2015;24(5):747-753. Available from: http://dx.doi.org/10.1016/j.jse.2014.12.024doi:10.1016/j.jse.2014.12.024
45. Verran J, Whitehead K. Factors affecting microbial adhesion to stainless steel and other materials used in medical devices. Int. J. Artif. Organs. 2005;28(11):1138-1145.
46. Wassmann T, Kreis S, Behr M, Buergers R. The influence of surface texture and wettability on initial bacterial adhesion on titanium and zirconium oxide dental implants. Int. J. Implant Dent. [Internet]. 2017;3(1):32. Available from: http://journalimplantdent.springeropen.eom/articles/10.1186/s40729-017 -0093-3doi:10.1186/s40729-017-0093-3
47. White SH, Wimley WC, Selsted ME. Structure, function, and membrane integration of defensins. Curr. Opin. Struct. Biol. 1995;5(4):521-527. doi:10.1016/0959-440X(95)80038-7
48. Whitehead KA, Colligon JS, Verran J. The production of surfaces of defined topography and chemistry for microbial retention studies, using ion beam sputtering technology. Int. Biodeterior. Biodegrad. 2004;54(2-3):143-151. doi:10.1016/j.ibiod.2004.03.010
49. Yamauchi M, Yamamoto K, Wakabayashi M, Kawano J. In vitro adherence of microorganisms to denture base resin with different surface texture. Dent. Mater. J. [Internet]. 1990;9(1):19-24. Available from: http://www.ncbi.nlm.nih.gov/entrez/query.fcgi?cmd=Retrieu9&db=PubMed&dopt=Citatio n&list_uids=2098207doi:10.4012/dmj.9.19
50. Zilow EP, Hauck W, Linderkamp O, Zilow G. Alternative pathway activation of the complement system in preterm infants with early onset infection. Pediatr. Res. 1997;41 (3):334-339. doi:10.1203/00006450-199703000-00005

## Claims

1. A Biomarker for diagnosis of low-grade infections in a human subject, wherein the biomarker is selected from a group consisting of complement factors.

2. Biomarker according to claim 1, wherein the biomarker is selected from a group consisting of complement factors C3, C5 and C9.

3. Biomarker according to any of claims 1 or 2, wherein the biomarker is complement factor C9.

4. Method for diagnosis of low-grade infections in a human subject of comprising the steps:
- providing a sample of the human subject,
- ex vivo detection of the biomarker according to claims 1 to 3 within the sample,
- correlation of the result with the low-grade infection disease state.

5. Method according to claim 4, wherein the detection of the biomarker is performed by immunohistochemically staining, enzyme-linked immunosorbent assay (ELISA), Polymerase-chain-reaction, Enzyme-Linked ImmunoSpot, Enzyme multiplied immunoassay technique, Immunofluorescence, radiobinding assay, radioimmunoassay (RIA), Western Blot or Fluorescence in situ hybridization (FISH).

6. Biomarker according to claims 1 to 3 for use in a method according claims 4 or 5.

7. Use of Biomarker according to claims 1 to 3 or a method according to claims 4 and 5 for diagnosis of low-grade infection in a human subject.

## Amended claims

### Amended claims in accordance with Rule 137(2) EPC.

1. A Biomarker for diagnosis of low-grade infections in a human subject tissue sample, wherein the biomarker is selected from a group consisting of complement factors, wherein the biomarker is selected from a group consisting of complement factors C3, C5 and C9, preferably the biomarker is complement factor C9.

2. Method for diagnosis of low-grade infections in a human subject of comprising the steps:
- providing a sample of the human subject, wherein the sample is a tissue sample
- ex vivo detection of the biomarker according to claim 1 within the sample,
- correlation of the result with the low-grade infection disease state.

3. Method according to claim 2, wherein the detection of the biomarker is performed by immunohistochemically staining, enzyme-linked immunosorbent assay (ELISA), Polymerase-chain-reaction, Enzyme-Linked ImmunoSpot, Enzyme multiplied immunoassay technique, Immunofluorescence, radiobinding assay, radioimmunoassay (RIA), Western Blot or Fluorescence in situ hybridization (FISH).

4. Biomarker according to claim 1 for use in a method according claims 2 or 3.

5. Use of Biomarker according to claim 1 or a method according to claims 2 or 3 for diagnosis of low-grade infection in a human subject tissue sample.
